# EUROPEAN PATENT APPLICATION

(11) **EP 3 865 122 A1**
(43) Date of publication of application: **18.08.2021**
(21) Application number: 20000065.1
(22) Date of filing: 11.02.2020
(51) Int. Cl.: A61K 9/127, A61K 31/7105, A61K 47/69, C12N 15/88

(54) **LIPID COMPOSITION AND USE THEREOF FOR DELIVERY OF A THERAPEUTICALLY ACTIVE AGENT TO ENDOTHELIUM**

(71) Applicant: Pantherna Therapeutics GmbH, 16761 Hennigsdorf (DE)
(72) Inventor: The designation of the inventor has not yet been filed
(74) Representative: Bohmann, Armin K.

(57) **Abstract**

The present invention is related to a composition comprising a lipid composition, wherein the lipid composition comprises a cationic lipid, a neutral lipid and a shielding lipid,
wherein the cationic lipid is selected from the group consisting of β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-Di-O-octadecenyl-3-trimethylammonium propane (preferably chloride salt)) and DC-cholesterol (3ß-[N-(N',N'-Dimethylaminoethane)-carbamoyl] cholesterol),
wherein the neutral lipid is a zwitterionic phospholipid selected from the group consisting of Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Dilauroyl-sn-glycero-3-phosphoethanolamine), DMPE ((1,2-Dimyristoyl-sn-glycero-3-phosphoethanolamine)), POPE ((1-Palmitoyl-2-oleoyl-sn-glycero-3-phosphoethanolamine)) and DSPE (1,2-Distearoyl-sn-glycero-3 -phosphoethanolamine),
wherein the shielding lipid is a PEGylated lipid selected from the group consisting methoxyPEG-DSPE ((1,2-Distearoyl-sn-glycero-3-phosphoethanolamine-N-[methoxy(polyethylene glycol)] (ammonium or sodium salt), methoxyPEG-DLG (1,2-Dilauroyl-rac-glycero-3-methoxypolyethylene glycol), methoxyPEG-DMG (1,2-Dimyristoyl-rac-glycero-3-methoxypolyethylene glycol), methoxyPEG-DPG (1,2-Dipalmitoyl-rac-glycero-3-methoxypolyethylene glycol), methoxyPEG-DSG (1,2-Distearoyl-rac-glycero-3-methoxypolyethylene glycol), methoxyPEG-c-DMA (N-[(Methoxy poly(ethylene glycol))carbamyl]-1,2-dimyristyloxlpropyl-3-amine), methoxyPEG-C8-ceramide (N-Octanoyl-sphingosine-1 - {succinyl[methoxy(polyethylene glycol)]}), methoxyPEG-C16-ceramide (N-Palmitoyl-sphingosine-1-{succinyl[methoxy(polyethylene glycol)]}), and
wherein the composition comprises an mRNA.

## Description

The present invention is related to a composition comprising a lipid composition; the composition comprising a lipid composition for use in a method for the treatment of a disease; use of the composition comprising a lipid composition for the manufacture of a medicament for the treatment and/or prevention of a disease; a pharmaceutical composition comprising a composition comprising a lipid composition; a kit comprising the composition comprising a lipid composition; and a method for the treatment and/or prevention of a disease, wherein the method comprises administering to a subject in need thereof an effective amount of the composition comprising a lipid composition.

Both molecular biology as well as molecular medicine heavily rely on the introduction of biologically active compounds into cells. Such biologically active compounds typically comprise, among others, DNA, RNA as well as peptides and proteins, respectively. The barrier which has to be overcome is typically a lipid bilayer which has a negatively charged outer surface. In the art, a number of technologies have been developed to penetrate the cellular membrane and to thus introduce the biologically active compounds. Some methods conceived for laboratory use, however, cannot be used in the medical field and are more particularly not suitable for drug delivery. For example, electroporation and ballistic methods known in the art, would, if at all, only allow a local delivery of biologically active compounds. Apart from said lipid bilayer cellular membranes also comprise transporter systems. Accordingly, efforts were undertaken to use this kind of transporter systems in order to transfer the biologically active compounds across the cell membrane. However, due to the specificity or cross-reactivity of such transporter systems, their use is not a generally applicable method.

A more generally applicable approach described in the art for transferring biologically active compounds into cells, is the use of viral vectors. However, viral vectors can be used only for transferring genes efficiently into some cell types; but they cannot be used to introduce chemically synthesized molecules into the cells.

An alternative approach was the use of so-called liposomes (Bangham, J. Mol. Biol. 13, 238-252). Liposomes are vesicles which are generated upon association of amphiphilic lipids in water. Liposomes typically comprise concentrically arranged bilayers of phospholipids. Depending on the number of layers liposomes can be categorized as small unilamelar vesicles, multilamelar vesicles and large multilamelar vesicles. Liposomes have proven to be effective delivery agents as they allow incorporating hydrophilic compounds into the aqueous intermediate layers, whereas hydrophobic compounds are incorporated into the lipid layers. It is well known in the art that both the composition of the lipid formulation as well as its method of preparation have an effect on the structure and size of the resultant lipid aggregates and thus on the liposomes. Liposomes are also known to incorporate cationic lipids.

Cationic lipids have, apart from being components of liposomes, also attracted considerable attention as they may as such be used for cellular delivery of biopolymers. Using cationic lipids any anionic compound can be encapsulated essentially in a quantitative manner due to electrostatic interaction. In addition, it is believed that the cationic lipids interact with the negatively charged cell membranes initiating cellular membrane transport. It has been found that the use of a liposomal formulation containing cationic lipids or the use of cationic lipids as such together with a biologically active compound requires a heuristic approach as each formulation is of limited use because it typically can deliver plasmids into some but not all cell types, usually in the absence of serum.

Charge and/or mass ratios of lipids and the biologically active compounds to be transported by them have turned out to be a crucial factor in the delivery of different types of said biologically active compounds. For example, it has been shown that lipid formulations suitable for plasmid delivery comprising 5,000 to 10,000 bases in size, are generally not effective for the delivery of oligonucleotides such as siRNA molecules, synthetic ribozymes or antisense molecules typically comprising about 10 to about 50 bases. In addition, it has recently been indicated that optimal delivery conditions for antisense oligonucleotides and ribozymes are different, even in the same cell type.

US patent 6,395,713 discloses cationic lipid-based compositions whereby the cationic lipid consists of a lipophilic group, a linker and a head group and the use of such compositions for transferring biologically active compounds into a cell.

International patent application WO 2005/105152 discloses another cationic lipid-based composition which proved to be particularly effective in the delivery of functional nucleic acid molecule such as siRNA molecules.

Depending on the disease to be treated and the drug to be delivered, there is a need for delivering the drug to specific organs or specific cell types. One such specific organ is lung and one such specific cell type is pulmonary endothelial cell. The targeting of lung and pulmonary endothelial cell is, for example, advantageous in the delivery of a drug for the treatment of a disease such as acute lung injury, acute respiratory distress syndrome, lung cancer, pulmonary metastasis, pulmonary hypertension and pulmonary artery hypertension.

A problem underlying the present invention is the provision of a means capable of delivering an agent, preferably a therapeutically active agent, more preferably a drug, to lung. A further problem underlying the present invention is the provision of a means capable of delivering an agent, preferably a therapeutically active agent, more preferably a drug, to lung tissue. A still further problem underlying the present invention is the provision of a means which is capable of delivering an agent, preferably a therapeutically active agent, more preferably a drug, to a pulmonary endothelial cell. In connection with each and any of these problems, the agent and drug, respectively, is preferably an mRNA.

Another problem underlying the present invention is the provision of a means for the treatment of a lung disease, preferably a lung disease which is selected from the group comprising acute lung injury, acute respiratory distress syndrome, lung cancer, pulmonary metastasis, pulmonary hypertension and pulmonary artery hypertension. In connection with this problem, the agent and drug, respectively, is preferably an mRNA.

Another problem underlying the present invention is the provision of a delivery vehicle as part of a means for the treatment of a lung disease, preferably a lung disease which is selected from the group comprising acute lung injury, acute respiratory distress syndrome, lung cancer, pulmonary metastasis, pulmonary hypertension and pulmonary artery hypertension. In connection with this problem, the agent and drug, respectively, is preferably an mRNA.

Another problem underlying the present invention is the provision of a pharmaceutical composition. Preferable, the pharmaceutical is suitable for the delivery of an agent, preferably a therapeutically active agent, more preferably a drug, to lung. A further problem underlying the present invention is the provision of a pharmaceutical composition suitable for the delivery of an agent, preferably a therapeutically active agent, more preferably a drug, to lung tissue. A still further problem underlying the present invention is the provision of a pharmaceutical composition suitable for the delivery of an agent, preferably a therapeutically active agent, more preferably a drug, to a pulmonary endothelial cell. In connection with each and any of these problems, the agent and drug, respectively, is preferably an mRNA.

Another problem underlying the present invention is the provision of a means which can be used in the manufacture of a medicament, wherein the medicament is suitable for or is for use in the treatment of lung disease, preferably a lung disease which is selected from the group comprising acute lung injury, acute respiratory distress syndrome, lung cancer, pulmonary metastasis, pulmonary hypertension and pulmonary artery hypertension. In connection with this problem, the agent and drug, respectively, is preferably an mRNA.

Another problem underlying the present invention is the provision of a method and/or prevention for the treatment of a disease, wherein the method comprises administering to a subject in need thereof an effective amount a composition comprising a therapeutically or pharmaceutically active agent, preferably a drug. A further problem underlying the present invention is the provision of a method for the treatment and/or prevention of a lung disease, wherein the method comprises administering to a subject in need thereof an effective amount a composition comprising a therapeutically or pharmaceutically active agent, preferably a drug. A still further problem underlying the present invention is the provision of a method for the treatment and/or prevention of a disease, preferably a lung disease, whereby the treatment comprises delivering a therapeutically or pharmaceutically active agent to the lung, preferably to a pulmonary endothelial cell. In connection with each and any of these problems, the agent and drug, respectively, is preferably an mRNA.

Another problem underlying the present invention is the provision of a kit. Preferably, the kit is suitable (a) for use in a method for the treatment and/or prevention of a disease, preferably a lung disease, (b) for use in a method of transferring a biologically active agent, a therapeutically active agent and/or or pharmaceutically active agent into a cell or across a membrane of a cell, whereby preferably such cell is a pulmonary endothelial cell, and/or (c) for use in the manufacture of a medicament, preferably a medicament for the treatment and/or prevention of a disease, more preferably a lung disease and most preferably a lung disease selected from the group comprising acute lung injury, acute respiratory distress syndrome, lung cancer, pulmonary metastasis, pulmonary hypertension and pulmonary artery hypertension. In connection with each and any of these problems, the agent and drug, respectively, is preferably an mRNA.

These and other problems are solved by the subject matter of the attached independent claims. Preferred embodiments may be taken from the attached dependent claims. These and other problems are also solved by the following embodiments.
Embodiment 1: A composition comprising a lipid composition, wherein the lipid composition comprises a cationic lipid, a neutral lipid and a shielding lipid,
   wherein the cationic lipid is selected from the group comprising β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-Di-O-octadecenyl-3-trimethylammonium propane (preferably as chloride salt)) and DC-cholesterol (3β-[N-(N',N'-Dimethylaminoethane)-carbamoyl]cholesterol),
   wherein the neutral lipid is a zwitterionic phospholipid selected from the group comprising Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Dilauroyl-sn-glycero-3-phosphoethanolamine), DMPE ((1,2-Dimyristoyl-sn-glycero-3-phosphoethanolamine)), POPE ((1-Palmitoyl-2-oleoyl-sn-glycero-3-phosphoethanolamine)) and DSPE (1,2-Distearoyl-sn-glycero-3-phospho), or
   an uncharged sterol lipid selected from a group comprising cholesterol and stigmasterol,
   wherein the shielding lipid is a PEGylated lipid selected from the group comprising methoxyPEG-DSPE ((1,2-Distearoyl-sn-glycero-3-phosphoethanolamine-N-[methoxy(polyethylene glycol)] (preferably as ammonium or sodium salt), methoxyPEG-DLG (1,2-Dilauroyl-rac-glycero-3-methoxypolyethylene glycol), methoxyPEG-DMG (1,2-Dimyristoyl-rac-glycero-3-methoxypolyethylene glycol), methoxyPEG-DPG (1,2-Dipalmitoyl-rac-glycero-3-methoxypolyethylene glycol), methoxyPEG-DSG (1,2-Distearoyl-rac-glycero-3-methoxypolyethylene glycol), methoxyPEG-c-DMA (N-[(Methoxy poly(ethylene glycol))carbamyl]-1,2-dimyristyloxlpropyl-3-amine), methoxyPEG-C8-ceramide (N-Octanoyl-sphingosine-1-{succinyl[methoxy(polyethylene glycol)]}), methoxyPEG-C 1 6-ceramide (N-Palmitoyl-sphingosine-1-{succinyl[methoxy(polyethylene glycol)]}), and
   wherein the composition comprises an mRNA.
Embodiment 2: A composition comprising a lipid composition, wherein the lipid composition comprises a cationic lipid, a neutral lipid and a shielding lipid,
   wherein the cationic lipid is selected from the group comrprising β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-Di-O-octadecenyl-3-trimethylammonium propane (preferably as chloride salt)) and DC-cholesterol (3B-[N-(N',N'-Dimethylaminoethane)-carbamoyl]cholesterol)
   wherein the neutral lipid is
   a zwitterionic phospholipid selected from the group comprising Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Dilauroyl-sn-glycero-3-phosphoethanolamine), DMPE ((1,2-Dimyristoyl-sn-glycero-3-phosphoethanolamine)), POPE ((1-Palmitoyl-2-oleoyl-sn-glycero-3-phosphoethanolamine)) and DSPE (1,2-Distearoyl-sn-glycero-3-phosphoethanolamine), or
   an uncharged sterol lipid selected from a group comprising cholesterol and stigmasterol, and
   wherein the shielding lipid is a PEGylated lipid selected from the group comprising methoxyPEG-DSPE ((1,2-Distearoyl-sn-glycero-3-phosphoethanolamine-N-[methoxy(polyethylene glycol)] (preferably as ammonium or sodium salt), methoxyPEG-DLG (1,2-Dilauroyl-rac-glycero-3-methoxypolyethylene glycol), methoxyPEG-DMG (1,2-Dimyristoyl-rac-glycero-3-methoxypolyethylene glycol), methoxyPEG-DPG (1,2-Dipalmitoyl-rac-glycero-3-methoxypolyethylene glycol), methoxyPEG-DSG (1,2-Distearoyl-rac-glycero-3-methoxypolyethylene glycol), methoxyPEG-c-DMA (N-[(Methoxy poly(ethylene glycol))carbamyl]-1,2-dimyristyloxlpropyl-3-amine), methoxyPEG-C8-ceramide (N-Octanoyl-sphingosine-l-{succinyl[methoxy(polyethylene glycol)]}) and methoxyPEG-C 16-ceramide (N-Palmitoyl-sphingosine-1 - {succinyl[methoxy(polyethylene glycol)]}), and
   and
   wherein the lipid composition forms particles, wherein the particle size is from 30 nm to 100 nm, preferably from about 30 nm to about 60 nm.
Embodiment 3: The composition of Embodiment 2, wherein the composition comprises an mRNA.
Embodiment 4: The composition of Embodiment 1, wherein the lipid composition forms particles, wherein the particle size is from about 30 nm to about 100 nm, preferably the particle size is from about 30 nm to about 60 nm.
Embodiment 5: The composition of any one of Embodiments 2 to 4, wherein the particles comprise an mRNA.
Embodiment 6: The composition of any one of Embodiments 1 to 5, wherein the particle size is determined by dynamic light scattering.
Embodiment 7: The composition of any one of Embodiments 1 to 6, wherein the zeta potential of the particles is about + 25 mV to about + 80 mV, preferably the zeta potential of the particles is about + 30 mV to about + 60 mV.
Embodiment 8: The composition of Embodiment 7, wherein the zeta potential is measured by Laser Doppler Electrophoresis measurement.
Embodiment 9: The composition of any one of Embodiments 1 and 3 to 8, wherein the mRNA forms part of the particles.
Embodiment 10: The composition of any one of Embodiments 1 and 2 to 9, wherein the ratio of the mass of the total lipid content of the composition to the mass of the mRNA of the composition is from about 2 to about 50, preferably from about 5 to 40, and more preferably from about 10 to about 30.
Embodiment 11: The composition of any one of Embodiments 1 to 10, wherein the composition is suitable for delivering a therapeutically active agent to an endothelial cell.
Embodiment 12: The composition of Embodiment 11, wherein the endothelial cell is a pulmonary endothelial cell.
Embodiment 13: The composition of any one of Embodiments 1 to 12, wherein the composition is suitable for delivering a therapeutically active agent to vasculature, preferably pulmonary vasculature, more preferably to human pulmonary vasculature.
Embodiment 14: The composition of any one of Embodiments 1 to 13, wherein the therapeutically active agent is an mRNA, preferably the therapeutically active agent is the mRNA comprised by the composition.
Embodiment 15: The composition of any one of Embodiments 1 and 2 to 14, wherein the mRNA forms a complex with the lipid composition.
Embodiment 16: The composition of any one of Embodiments 1 to 15, wherein the composition comprises a carrier, preferably a pharmaceutically acceptable carrier.
Embodiment 17: The composition of Embodiment 16, wherein the carrier is selected from the group comprising water, an aqueous solution, preferably an isotonic aqueous solution, a salt solution, preferably an isotonic salt solution, a buffer solution, preferably an isotonic buffer solution and a water miscible solvent.
Embodiment 18: The composition of Embodiment 17, wherein the carrier is water miscible solvent and wherein the water miscible solvent is selected from the group comprising ethanol, acetone, 1-butanol, 2-butanol, tert.-butanol, 3-methyl-1-butanol, 2-methyl-1-propanol, 1-propanol, 2-propanol, dimethylsulfoxide, preferably the water miscible solvent is selected from the group comprising ethanol, tert.-butanol and 1-butanol.
Embodiment 19: The composition of any one of Embodiments 16 to 18, wherein the carrier is an aqueous sucrose solution, preferably a 270 mM aqueous sucrose solution or a 270 mM sucrose solution in a buffer, preferably the buffer is a 10 mM TRIS buffer pH 7.4.
Embodiment 20: The composition of any one of Embodiments 1 to 19, wherein the molecular weight of the PEG of the PEGylated lipid is from about 750 Da to about 5000 Da, preferably from about 1500 Da to 3000 Da.
Embodiment 21: The composition of any one of Embodiments 1 to 20, wherein the molar ratio of the lipids in the lipid composition is
   - from about 20 mol-% to about 80 mol-% of the cationic lipid,
   - from about 10 mol-% to about 70 mol-% of the neutral lipid, and
   - from about 1 mol-% to 10 mol-% of the PEGylated lipid,
   wherein the overall lipid content is 100 %.
Embodiment 22: The composition of Embodiment 21, wherein the molar ratio of the lipids in the lipid composition is
   - from about 35 mol-% to about 65 mol-% of the cationic lipid,
   - from about 35 mol-% to about 65 mol-% of the neutral lipid, and
   - from about 1 mol-% to 5 mol-% of the PEGylated lipid,
   wherein the overall lipid content is 100 %.
Embodiment 23: The composition of any one of Embodiments 1 to 22, wherein cationic lipid is β-(L-Arginyl)-L-2,3-diamino propionic acid-N-palmityl-N-oleyl-amide or L-Arginyl-β-alanine-N-palmityl-N-oleyl-amide.
Embodiment 24: The composition of any one of Embodiments 1 to 23, preferably claim 23, wherein the neutral lipid is the phospholipid Diphytanoyl-PE.
Embodiment 25: The composition of any one of Embodiments 1 to 24, preferably claims 23 and 24, more preferably claim 24, wherein the PEGylated lipid is methoxyPEG2000-DSPE.
Embodiment 26: The composition of any one of Embodiments 1 to 25, wherein the molar ratio of the lipid composition is
   - 50 mol-% of the cationic lipid, wherein the cationic lipid is β-(L-Arginyl)-L-2,3-diamino propionic acid-N-palmityl-N-oleyl-amide or L-Arginyl-β-alanine-N-palmityl-N-oleyl-amide,
   - 49 mol-% of the neutral lipid, wherein the neutral lipid is Diphytanoyl-PE (1,2-Diphytanoyl-sn-glycero-3-phosphoethanolamine), and
   - 1 mol-% of the shielding lipid, wherein the shielding lipid is mPEG-2000-DSPE (1,2-distearoyl-sn-glycero-3-phosphoethanolamine-N-[methoxy(polyethylene glycol)-2000] (preferably as sodium salt)).
Embodiment 27: The composition of any one of Embodiments 1 to 26 for use in a method for the treatment and/or prevention of a disease.
Embodiment 28: The composition for use of Embodiment 27, wherein the treatment and/or prevention comprises administration of the composition or the mRNA of the composition to an endothelial cell, preferably an endothelial cell of vasculature, more preferably an endothelial cell of lung vasculature.
Embodiment 29: The composition for use of Embodiment 28, wherein the endothelium is human endothelium.
Embodiment 30: The composition for use of any one of Embodiments 28 to 29, wherein the disease is a disease where a target molecule involved in the pathological mechanism underlying the disease is present in an endothelial cell of lung vasculature and the provision of the mRNA provides for a therapeutic effect.
Embodiment 31: The composition for use of any one of Embodiments 27 to 30, wherein the disease is selected from the group comprising acute respiratory distress syndrome, acute lung injury, lung cancer, pulmonary metastasis, pulmonary hypertension and pulmonary artery hypertension.
Embodiment 32: The composition of any one of Embodiments 27 to 31, wherein the composition is for intravenous, intramuscular, subcutaneous, intradermal, intravitreal, intrathecal, perivascular, intranasal administration, and/or for administration by inhalation, preferably, the composition is for intravenous administration.
Embodiment 33: Use of a composition of any one of Embodiments 1 to 26, in the manufacture of a medicament for the treatment and/or prevention of a disease.
Embodiment 34: Use of Embodiment 33, wherein treatment and/or prevention of a disease comprises administration of the composition or the mRNA of the composition to an endothelial cell, preferably an endothelial cell of vasculature, more preferably an endothelial cells of lung vasculature.
Embodiment 35: Use of Embodiment 34, wherein the endothelium is human endothelium.
Embodiment 36: Use of any one of Embodiments 34 to 35, wherein the disease is a disease where a target molecule involved in the pathological mechanism underlying the disease is present in an endothelial cell of lung vasculature and the provision of the mRNA provides for a therapeutic effect.
Embodiment 37: Use of any one of Embodiments 33 to 36, wherein the disease is selected from the group comprising acute respiratory distress syndrome, acute lung injury, lung cancer, pulmonary metastasis, pulmonary hypertension and pulmonary artery hypertension.
Embodiment 38: Use of any one of Embodiments 33 to 37, wherein the medicament is for intravenous, intramuscular, subcutaneous, intradermal, intravitreal, intrathecal, perivascular, intranasal administration, and/or for administration by inhalation, preferably the medicament is for intravenous administration.
Embodiment 39: A pharmaceutical composition comprising a composition of any one of Embodiments 1 to 26 and a pharmaceutically active agent.
Embodiment 40: The pharmaceutical composition of Embodiment 39, wherein the mRNA of the composition of any one of claims 1 to 26 is a therapeutically active agent.
Embodiment 41: The pharmaceutical composition of any one of Embodiments 39 to 40, for use in the treatment and/or prevention of a disease, wherein the disease is preferably selected from the group comprising acute respiratory distress syndrome, acute lung injury, lung cancer, pulmonary metastasis, pulmonary hypertension and pulmonary artery hypertension.
Embodiment 42: A kit comprising a composition of any one of Embodiments 1 to 26 and instructions of use.
Embodiment 43: A method for the treatment and/or prevention of a disease, wherein the method comprises administering to a subject in need thereof a composition of any one of Embodiments 1 to 26, wherein the mRNA of the composition is therapeutically active, preferably, the mRNA is suitable for the treatment of the disease.
Embodiment 44: The method of Embodiments 43, wherein the disease is preferably selected from the group comprising acute respiratory distress syndrome, acute lung injury, lung cancer, pulmonary metastasis, pulmonary hypertension and pulmonary artery hypertension.
Embodiment 45: The method of any one of Embodiments 43 to 44, wherein the subject is selected from the group comprising man, mouse, rat, rabbit, hamster, guinea pig, monkey, dog, cat, pig, sheep, goat, cow and horse, preferably the subject is man.
Embodiment 46: A method for preparing a composition of any one of Embodiments 1 to 26, wherein the method comprises mixing a solution comprising the lipid components of the lipid composition with a solution comprising the mRNA, wherein the mixing is an in-line mixing.
Embodiment 47: The method of Embodiment 46, wherein the mixing is a non-turbulent and diffusion-based mixing, preferably a rapid non-turbulent and diffusion-based mixing.
Embodiment 48: The method of any one of Embodiments 46 to 47, wherein the mixing is a microfluidic mixing, preferably a microfluidic mixing using a microfluidic mixing device.
Embodiment 49: The method of Embodiment 48, wherein the microfluidic mixing device is selected from the group comprising a staggered herringbone mixer, a microfluidic hydrodynamic mixing device or a Dean Vortex bifurcating mixing device.
Embodiment 50: The method of any one of Embodiments 46 to 49, wherein the solution comprising the lipid component comprises the components of the lipid composition in a water miscible organic solvent.
Embodiment 51: The method of Embodiment 50, wherein the water miscible organic solvent is selected from the group comprising ethanol, acetone, 1-butanol, 2-butanol, tert.-butanol, 3-methyl-1-butanol, 2-methyl-1-propanol, 1-propanol, 2-propanol, dimethylsulfoxide, preferably the water miscible solvent is selected from the group comprising ethanol, tert.-butanol and 1-butanol.
Embodiment 52: The method of any one of Embodiments 46 to 51, wherein the solution comprising the mRNA comprises the mRNA in water or an aqueous buffer.
Embodiment 53: The method of Embodiment 52, wherein the aqueous buffer is a citrate buffer, acetate buffer, TRIS buffer (2-Amino-2-(hydroxymethyl)propane-1,3-diol) or HEPES buffer (2-[4-(2-hydroxyethyl)piperazin-1-yl]ethanesulfonic acid).
Embodiment 54: The method of any one of Embodiments 46 to 53, wherein the volumetric mixing ratio of the solution comprising the mRNA to the solution comprising the lipid components of the lipid composition is from about 1:1 to about 6:1, preferably from about 2:1 to about 4:1.
Embodiment 55: The method of any one of Embodiments 46 to 54, wherein the organic solvent is removed from the mixture after the mixing.
Embodiment 56: The method of Embodiment 55, wherein the organic solvent is removed from the mixture after the mixing by dialysis or tangential flow filtration.
Embodiment 57: The method of Embodiment 56, wherein the ultrafiltration membrane used in tangential flow filtration and/or dialysis has a molecular weight cut-off of from about 1,500 Da to about 500,000 Da, preferably of from about 1,500 Da to about 100,000 Da and more preferably of from about 1,500 Da to about 30,000 Da.
Embodiment 58: The method of any one of Embodiments 46 to 57, wherein the reaction mixture is concentrated after the mixing, preferably after removing the organic solvent.
Embodiment 59: The method of Embodiment 58, wherein the reaction mixture is concentrated by tangential flow filtration.

The present inventors have surprisingly found that a composition comprising a lipid composition, wherein the lipid composition comprises a cationic lipid, a neutral lipid and a shielding lipid,
wherein the cationic lipid is selected from the group comprising β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-Di-O-octadecenyl-3-trimethylammonium propane (chloride salt)) and DC-cholesterol (3β-[N-(N',N'-Dimethylaminoethane)-carbamoyl]cholesterol),
wherein the neutral lipid is
a zwitterionic phospholipid selected from the group comprising Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Dilauroyl-sn-glycero-3-phosphoethanolamine), DMPE ((1,2-Dimyristoyl-sn-glycero-3-phosphoethanolamine)), POPE ((1-Palmitoyl-2-oleoyl-sn-glycero-3-phosphoethanolamine)) and DSPE (1,2-Distearoyl-sn-glycero-3-phosphoethanolamine), or
an uncharged sterol lipid selected from a group comprising cholesterol and stigmasterol,
wherein the shielding lipid is a PEGylated lipid selected from the group comprising methoxyPEG-DSPE ((1,2-Distearoyl-sn-glycero-3-phosphoethanolamine-N-[methoxy(polyethylene glycol)] (ammonium or sodium salt), methoxyPEG-DLG (1,2-Dilauroyl-rac-glycero-3-methoxypolyethylene glycol), methoxyPEG-DMG (1,2-Dimyristoyl-rac-glycero-3-methoxypolyethylene glycol), methoxyPEG-DPG (1,2-Dipalmitoyl-rac-glycero-3-methoxypolyethylene glycol), methoxyPEG-DSG (1,2-Distearoyl-rac-glycero-3-methoxypolyethylene glycol), methoxyPEG-c-DMA (N-[(Methoxy poly(ethylene glycol))carbamyl]-1,2-dimyristyloxlpropyl-3-amine), methoxyPEG-C8-ceramide (N-Octanoyl-sphingosine-1 - {succinyl[methoxy(polyethylene glycol)]}), methoxyPEG-C 16-ceramide (N-Palmitoyl-sphingosine-1-{succinyl[methoxy(polyethylene glycol)]})
is particularly effective in targeting an endothelial cell, preferably an endothelial cell of vasculature and more preferably an endothelial cell of lung vasculature.

It is acknowledged by a person skilled in the art that the vascular endothelium is the inner-most structure that coats the interior walls of arteries, capillaries and veins. Endothelial cells (EC) were described to anchor to an 80-nm-thick basal lamina (BL). Both EC and BL constitute the vascular intima, establishing a hemocompatible surface, estimated a total combined surface area of 3000-6000 m2 in the human body, comprising 1 to 6 × 1013 EC (Krüger-Genge, A., Blocki, A., Franke, R.-P. & Jung, F. Vascular Endothelial Cell Biology: An Update. Int J Mol Sci 20, 4411 (2019).)

Additionally, the present inventors have surprisingly found that such targeting allows delivery of mRNA to said endothelial cell, whereby such mRNA is part of the composition of the present invention, preferably such mRNA is associated with the lipid composition of the composition of the invention. More preferably, such mRNA is part of particles formed by the lipid composition of the composition of the present invention.

Again, without wishing to be bound by any theory, the present inventors assume that said delivery of mRNA to endothelial cells is, in part, caused by the size distribution of the particles formed by the lipid composition of the composition of the present invention.

The above finding is insofar surprising as it contrasts typical systemic behavior of cationic lipid nano-complexes which generally show prevalent gene knockdown in liver tissue with only transient accumulation of siRNA in the lung (Polach, KJ et al. (2012), Mol Ther 20: 91-100; Schroeder, A et al. (2010), J Intern Med 267: 9-21; Tao, W et al. (2010), Mol Ther 18: 1657-1666).

As preferably used herein, a delivery agent or a delivery vehicle is a composition comprising the lipid composition of the invention. As also preferably used herein, a delivery agent or a delivery vehicle is a composition of the invention. A delivery agent or a delivery vehicle as preferably used herein is an agent or a vehicle such as a composition which is suitable to deliver a compound to a structure; preferably such structure is an organ, tissue or cell; more preferably such structure is an organ, tissue or cell. In a preferred embodiment such compound is a therapeutically active agent, a biologically active agent or a pharmaceutically active agent.

As preferably used herein, a therapeutically active agent is a compound which is suitable to elicit in a host organism a therapeutic or therapeutically beneficial effect. In a preferred embodiment, the therapeutically active agent is an mRNA.

In connection with the instant invention, therapy and treatment, respectively, also encompasses prevention. In accordance therewith, a therapeutically active agent is, in an embodiment, also an agent which is active in prevention of a disease. In an alternative embodiment, a therapeutically active agent is not active in the prevention of a disease.

The composition of the present invention comprises a cationic lipid. It will be acknowledged by a person skilled in the art that any of the NH or NH2 group(s) present in said lipid are, preferably, present in a protonated form. Typically, any positive charge of said lipid is compensated by the presence of an anion. Such anion can be a monovalent or polyvalent anion. Preferred anions are halides, acetate and trifluoroacetate. Halides as used herein are preferably chlorides, fluorides, iodides and bromides. Most preferred are chlorides. Upon association of the cationic lipid and the therapeutically or pharmaceutically or biologically active compound to be transferred into a cell, the halide anion is replaced by the said active compound which preferably exhibits one or several negative charges, although it has to be acknowledged that the overall charge of the biologically active compound is not necessarily negative. The same considerations are equally applicable to the other compounds of the composition of the invention. In case such compound is an anionic compound or bears one or several negative charges such negative charges may be compensated by the presence of a cation. Such cation can be a monovalent or polyvalent cation. Preferred cations are ammonium, sodium or potassium.

The sterol compound of the composition of the invention can be either synthetic or be obtained from natural sources such as sheep wool or plants.

The PEGylated lipid of the composition of the invention is available from commercial sources such as NOF Corporation, Japan; Avanti Polar Lipids, US; or Cordon Pharma, Switzerland.

In an embodiment, compound β-(L-Arginyl)-L-2,3-diamino propionic acid-N-palmityl-N-oleyl-amide) is of the following formula:

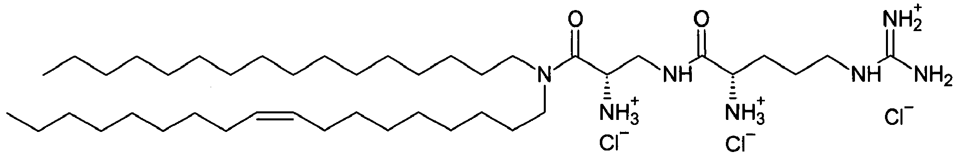

In an embodiment, compound L-Arginyl-β-alanine-N-palmityl-N-oleyl-amide is of the following formula:

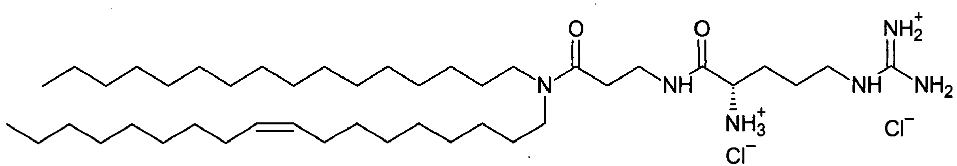

In an embodiment, compound DOTAP (N-[1-(2,3-Dioleoyloxy)propyl]-N,N,N-trimethylammonium methyl-chloride) is of the following formula:

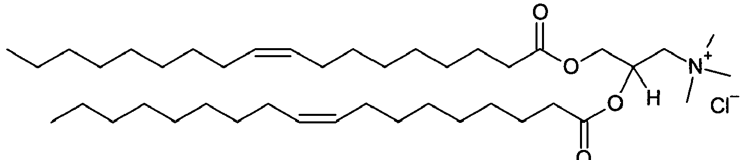

In an embodiment, compound DOTMA (1,2-Di-O-octadecenyl-3-trimethylammonium propane (preferably as chloride salt)) is of the following formula:

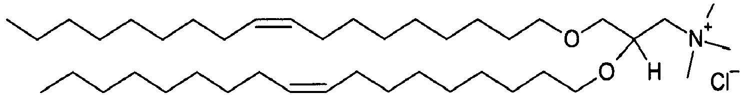

In an embodiment, compound DC-Cholesterol (3β-[N-(N',N'-Dimethylaminoethane)-carbamoyl]cholesterol hydrochloride) is of the following formula:

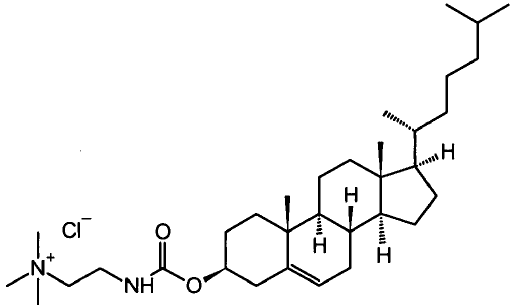

In an embodiment, compound DPhyPE (1,2-(7R,11R) Diphytanoyl-sn-glycero-3-phosphoethanolamine) is of the following formula:

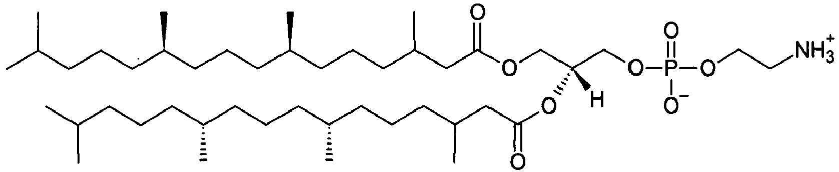

In an embodiment, compound DOPE (1,2-Dioleoyl-sn-glycero-3-phosphoethanolamine) is of the following formula:

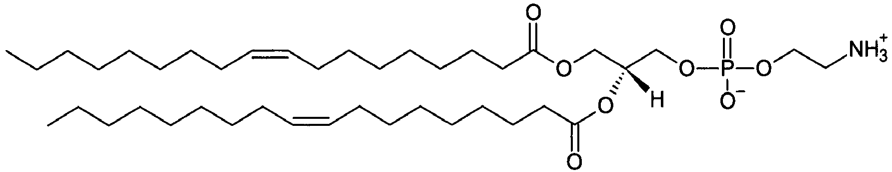

In an embodiment, compound DLPE (1,2-Dilauroyl-sn-glycero-3-phosphoethanolamine) is of the following formula:

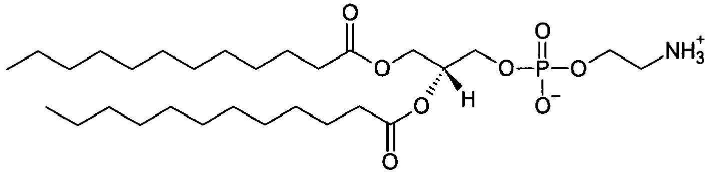

In an embodiment, compound DMPE (1,2-Dimyristoyl-sn-glycero-3-phosphoethanolamine) is of the following formula:

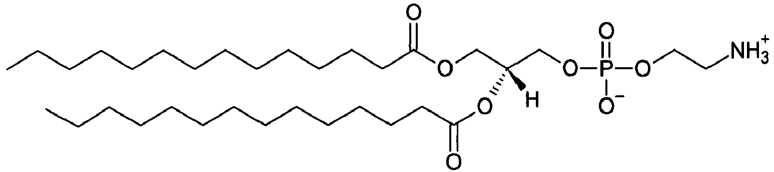

In an embodiment, compound POPE ((1-Palmitoyl-2-oleoyl-sn-glycero-3-phosphoethanolamine) is of the following formula:

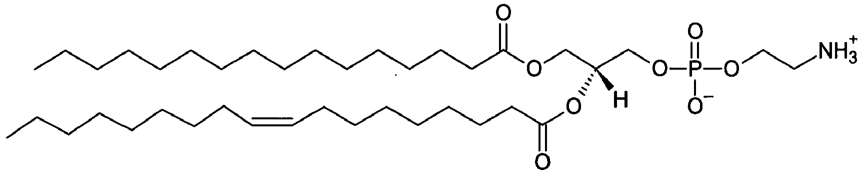

In an embodiment, compound DSPC (1,2-Distearoyl-sn-glycero-3-phosphoethanolamine) is of the following formula:

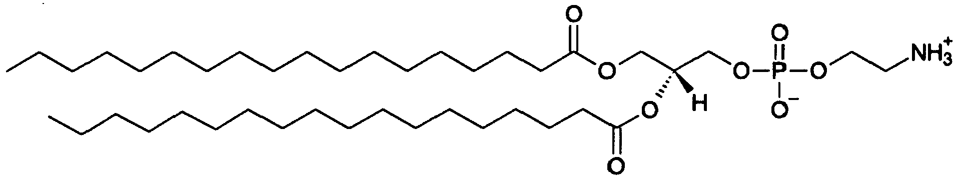

In an embodiment, compound cholesterol is of the following formula:

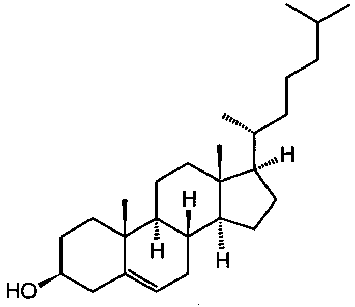

In an embodiment, compound Stigmasterol (Stigmasta-5,22-dien-3-ol) is of the following formula:

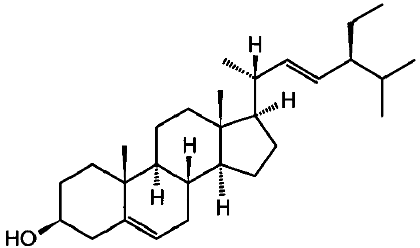

In an embodiment, compound mPEG-500-DSPE (1,2-Distearoyl-sn-glycero-3-phosphoethanolamine-N-[methoxy(polyethylene glycol)-500] (preferably as an ammonium salt) is of the following formula:

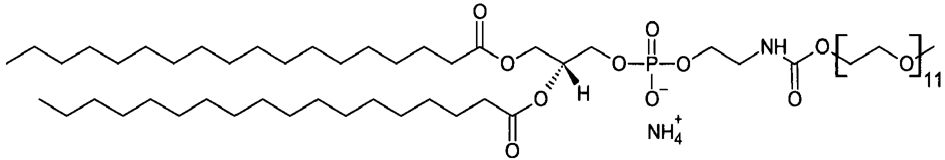

In an embodiment, compound mPEG-looo-DSPE (1,2-Distearoyl-sn-glycero-3-phosphoethanolamine-N-[methoxy(polyethylene glycol)-1000] (preferably as ammonium salt) is of the following formula:

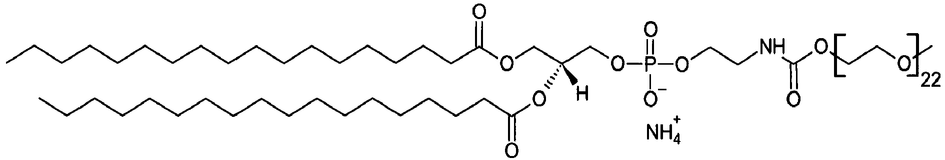

In an embodiment, compound mPEG-2ooo-DSPE (1,2-Distearoyl-sn-glycero-3-phosphoethanolamine-N-[methoxy(polyethylene glycol)-2000] (preferably as ammonium salt) is of the following formula:

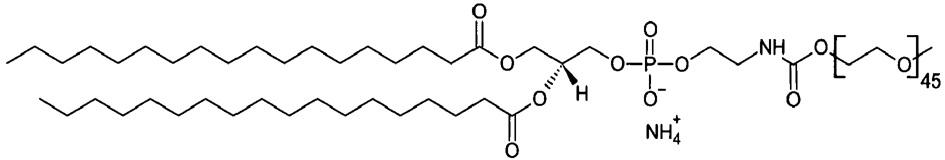

In an embodiment, compound mPEG-5ooo-DSPE (1,2-Distearoyl-sn-glycero-3-phosphoethanolamine-N-[methoxy(polyethylene glycol)-5000] (preferably as ammonium salt) is of the following formula:

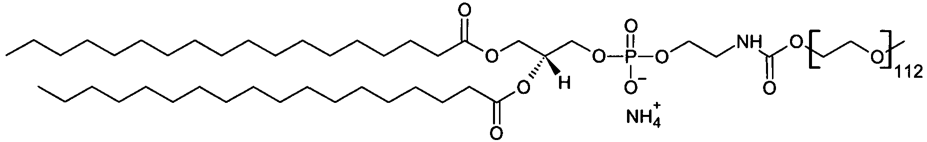

In an embodiment, compound mPEG-DSPE (1,2-Distearoyl-sn-glycero-3-phosphoethanolamine-N-[methoxy(polyethylene glycol)] (preferably as ammonium salt) is of the following formula:

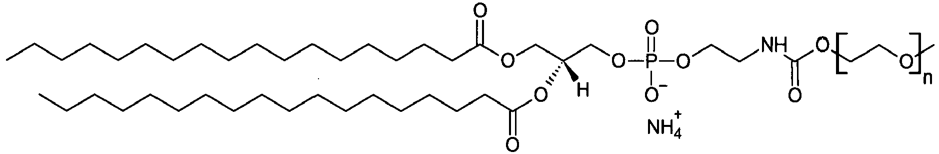

In an embodiment, compound mPEG-2000-DPG (1,2-Dipalmitoyl-rac-glycero-3-methoxypolyethylene glycol-2000) is of the following formula:

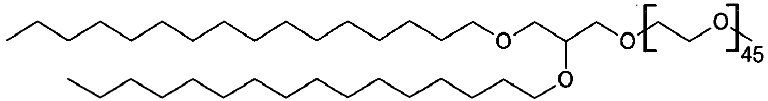

In an embodiment, compound mPEG-2000-DMG (1,2-Dimyristoyl-rac-glycero-3-methoxypolyethylene glycol-2000) is of the following formula:

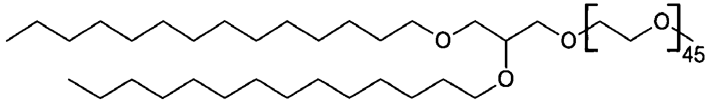

In an embodiment, compound mPEG-2000-DLG (1,2-Dilauroyl-rac-glycero-3-methoxypolyethylene glycol-2000) is of the following formula:

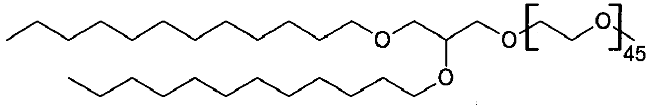

In an embodiment, compound mPEG-2000-C8-Ceramide (N-Octanoyl-sphingosine-1-{succinyl[methoxy(polyethylene glycol)2000]}) is of the following formula:

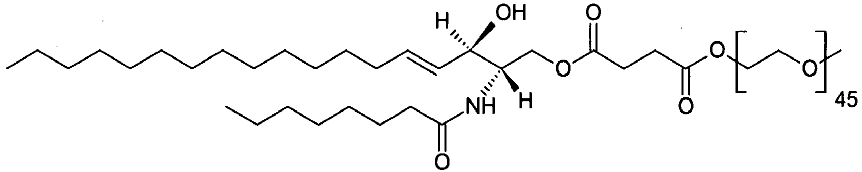

In an embodiment, compound mPEG-2000-C16-Ceramide (N-Palmitoyl-sphingosine-1-{succinyl[methoxy(polyethylene glycol)2000]}) is of the following formula:

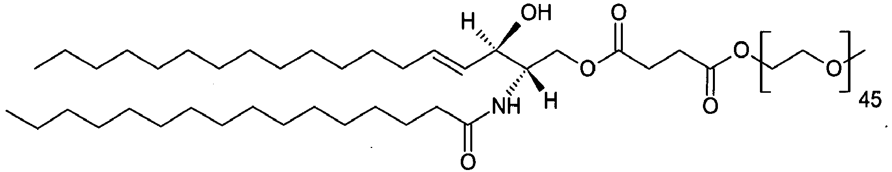

In an embodiment, compound mPEG-2000-C-DMA (N-[(Methoxy poly(ethylene glycol)2000)carbamyl]-1,2-dimyristyloxlpropyl-3-amine) is of the following formula:

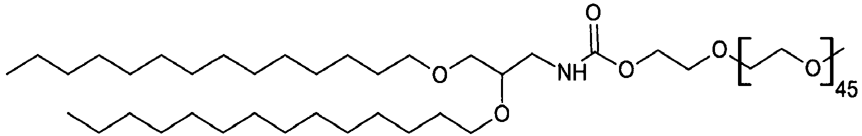

In an embodiment, particle size of the particles formed by the lipid composition of the composition of the invention is determined by Dynamic Light Scattering using a Zetasizer Ultra (Malvern Panalytical Ltd, Malvern, UK). All measurements are preferable carried out in an aqueous buffer as dispersant, which is a 270 mM sucrose solution buffered with 10 mM TRIS at pH 7.4. Dynamic Light Scattering is, for example, described in more detail in Stetefeld, J., McKenna, S. A. & Patel, T. R. "Dynamic light scattering: a practical guide and applications in biomedical sciences". Biophys Rev 8, 409-427 (2016); and Thomas, J. C. "The determination of log normal particle size distributions by dynamic light scattering". Journal of Colloid and Interface Science 117, 187-192 (1987); the disclosure of which is herein incorporated by reference.

More specifically, i.e. in a more preferred embodiment, particle size is determined as "Intensity Mean Peak" which is calculated by the measurement and analysis software ZX Explorer (Malvern Panalytical Ltd, Malvern, UK). The "Intensity Mean Peak" of the composition of the invention comprising an mRNA according to the Dynamic Light Scattering measurements is within a range of about 30 nm to about 100 nm and preferably within a range of about 30 nm to about 60 nm.

In an embodiment, the zeta potential of the particles formed by the lipid composition of the composition of the invention is determined by Laser Doppler Electrophoresis using a Zetasizer Ultra (Malvern Panalytical Ltd, Malvern, UK). All measurements are preferably carried out in an aqueous buffer as dispersant, which is a 270 mM sucrose solution buffered with 10 mM TRIS at pH 7.4. Laser Doppler Electrophoresis is, for example, described in more detail in Clogston, J. D. & Patri, A. K. in "Characterization of Nanoparticles Intended for Drug Delivery" 697, 63-70 (Humana Press, 2011); and Sze, A., Erickson, D., Ren, L. & Li, D. "Zeta-potential measurement using the Smoluchowski equation and the slope of the current-time relationship in electroosmotic flow", Journal of Colloid and Interface Science 261, 402-410 (2003), the disclosure of which is incorporated herein by reference.

More specifically, i.e. in a more preferred embodiment, zeta potential is determined as "Zeta Mean Peaks". Both expressions are calculated by the measurement and analysis software ZX Explorer (Malvern Panalytical Ltd, Malvern, UK). The "Zeta Mean Peak" of the composition of the invention comprising an mRNA according to the Laser Doppler Electrophoresis measurements is in a range of about +25 mV to about +80 mV and preferably in a range of about +30 mV to about + 60 mV.

In an embodiment and as preferably used herein, mRNA is a polynucleotide preferably comprising about 750 to about 5000 nucleotides, more preferably comprising about 1000 to 3000 nucleotides. In an embodiment thereof, the mRNA is a single-stranded nucleic acid, whereby some stretches thereof may be part of a or several double-stranded structure(s) within the polynucleotide. Such double-stranded structure is typically formed by two or more stretches of the polynucleotide the nucleotide sequence of which is at least partially complementary to each other. The double-stranded structure is preferable formed or stabilized by Watson-Crick base pairing.

In a further embodiment, the mRNA shows a very basic design in eukaryotic cells and typically comprises, in 5'-> 3' direction, a Cap structure, a 5' untranslated region (5' UTR), a coding sequence typically starting with a AUG codon attached to a coding sequence (CDS) terminating with a stop codon, a 3' untranslated region (3' UTR) and a poly-A-tail.

In accordance with the present invention, the mRNA, in an embodiment, is a therapeutically active agent. Useful in the treatment and/or prevention of a disease. In principle, the administered mRNA sequence can cause a cell to make a protein, which in turn could directly treat a disease or could function as a vaccine; more indirectly the protein could interfere with an element of a pathway in such way that the pathway is either inhibited or stimulated, thereby treating or ameliorating a disease.

In an embodiment to the present invention, the mRNA is different from a recombinant nucleic acid construct comprising in 5'-> 3' direction
- a 5' UTR,
- a coding region coding for an effector molecule, and
- a 3' UTR,
wherein the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for vWF or a derivative thereof having a nucleotide identity of at least 85 %, a 3' UTR of a gene coding for MCP-1 or a derivative thereof having a nucleotide identity of at least 85 %, , a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

In an embodiment to the present invention, the mRNA is recombinant nucleic acid construct comprising in 5'-> 3' direction
- a 5' UTR,
- a coding region coding for an effector molecule, and
- a 3' UTR,
wherein the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for vWF or a derivative thereof having a nucleotide identity of at least 85 %, a 3' UTR of a gene coding for MCP-1 or a derivative thereof having a nucleotide identity of at least 85 %, , a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell,
wherein the effector molecule is a mutant effector molecule, wherein the mutant effector molecule is a gain of function mutant effector molecule and/or a hypermorphic mutant effector molecule, and
wherein the recombinant nucleic acid construct is different from a wild type mRNA coding for the effector molecule.

The composition of the invention and particularly the lipid composition of the invention may comprise, in an embodiment, a carrier. Such carrier is preferably a liquid carrier. Preferred liquid carriers are aqueous carriers and non-aqueous carriers. Preferred aqueous carriers are water, an aqueous salt solution, an aqueous buffer system, more preferably the buffer system and/or the aqueous salt solution have a physiological buffer strength and physiological salt concentration(s). Preferred non-aqueous carriers are solvents, preferably organic solvents such as ethanol, tert.-butanol. Without wishing to be bound by any theory, any water miscible organic solvent can, in principle, be used. It is to be acknowledged that the composition, more particularly the lipid composition can thus be present as or form liposomes; when contacted with overall negatively charged compounds, preferably compounds to be delivered by the lipid composition of the invention and mRNA in particular and/or the composition of the invention, the lipid composition of the invention and the composition of the invention form lipoplexes, i.e. a complex that is formed by the electrostatic interaction and the entropic effect based on the release of counter ions and water when a polyanion such as a nucleic acid molecule interact with a cationic lipid or a lipid system that contains beside other lipid components at least one cationic lipid component.

In a further embodiment, the lipid composition of the invention and/or the composition of the invention is present as a lyophilized composition. The thus lyophilized composition allows effective long-term storage of the composition at room temperature.

In accordance with the present invention, a pharmaceutical composition comprises the composition of the present invention. The pharmaceutical composition of the invention comprises a pharmaceutically active compound and optionally a pharmaceutically acceptable carrier. Such pharmaceutically acceptable carrier may, preferably, be selected from the group of carriers as defined herein in connection with the composition according to the present invention. It will be understood by those skilled in the art that any composition as described herein may, in principle, be also used as a pharmaceutical composition provided that its ingredients and any combination thereof is pharmaceutically acceptable. A pharmaceutical composition comprises a pharmaceutically active compound. Such pharmaceutically active compound is, in an embodiment, the mRNA which is encompassed or comprised by the composition of the present invention.

It is within the ordinary skill of a person of the art that the composition, particularly the pharmaceutical composition according to the present invention can be used for various forms of administration, whereby the composition is to be adapted to such forms of administration.

The method of the present invention for preparing the composition of the present invention comprising an mRNA relies on mixing a solution comprising the lipid components of the lipid composition with a solution comprising the mRNA, wherein the mixing is an in-line mixing. The mixing step is performed by the application of a microfluidic mixing device and can particularly either be done by the use of a staggered herringbone mixer device, a Dean Vortex bifurcating mixing device or a microfluidic hydrodynamic mixing device. These devices allow due to their special designed micro channels a rapid, non-turbulent and diffusion based mixing processes. A staggered herringbone mixer and its use in the preparation of particles as preferably contained in the composition of the invention, is, for example, described in Zhigaltsev, I. V. et al. "Bottom-Up Design and Synthesis of Limit Size Lipid Nanoparticle Systems with Aqueous and Triglyceride Cores Using Millisecond Microfluidic Mixing". Langmuir 28, 3633-3640 (2012), the disclosure of which is incorporated herein by reference. Microfluidic hydrodynamic mixing and its use in the preparation of particles as preferably contained in the composition of the invention, is, for example, described in Krzysztoń, R. et al. "Microfluidic self-assembly of folate-targeted monomolecular siRNA-lipid nanoparticles." Nanoscale 9, 7442-7453 (2017), the disclosure of which is incorporated herein by reference. Finally, Dean Vortex bifurcating mixing and its use in the preparation of particles as preferably contained in the composition of the invention, is, for example, described in Chen, J. J., Chen, C. H. & Shie, S. R. "Optimal designs of staggered dean vortex micromixers." Int J Mol Sci 12, 3500-3524 (2011), the disclosure of which is incorporated herein by reference.

The instant invention is further illustrated by the following Examples from which further features, embodiment and advantages of the invention may be taken.

### Example 1: mRNA Formulation in cationic lipid nanoparticles (LNPs) for in vivo applications by intravenous administration

For in vivo experiments mRNA-LNPs are prepared in a formulation process with β-(L-Arginyl)-L-2,3-diamino propionic acid-N-palmityl-N-oleyl-amide as cationic lipid. Alternatively, the cationic lipid L-Arginyl-β-alanine-N-palmityl-N-oleyl-amide can be used in an identical procedure to prepare mRNA-LNPs.

Lipids are dissolved in ethanol at appropriate molar ratios (e.g. 50:49:1 β-(L-arginyl)-L-2,3-diamino propionic acid-N-palmityl-N-oleyl-amide: DPyPE: mPEG-2000-DSPE). The lipid mixture is combined with an aqueous solution of mRNA at a volume ratio of 2:1 (aqueous:ethanol) using a microfluidic mixer (NanoAssemblr®; Precision Nanosystems, Vancouver, BC) and flow rates of 18 ml/min. Similarly, LNP formulations can be obtained using citrate or acetate buffered mRNA solutions (pH 3-4). The final total lipid to mRNA mass ratio is 28.

After the mixing process, the formulations are dialyzed against 10 mM HEPES or TRIS buffered isotonic Sucrose solution using 3.5 kDa MWCO Slide-A-Lyzer Dialysis Cassettes (Thermo Fisher Scientific) for at least 18 hours at 4 °C. Instead of Sucrose, other sugars like Trehalose or Glucose can be equally used within the formulation process.

Subsequently, the formulations are tested for particle size (Zetasizer Ultra instrument (Malvern Instruments Ltd, Malvern, UK), RNA encapsulation (Quant-iT RiboGreen RNA Assay Kit following manufacturer's (Thermo Fisher Scientific) protocol), and endotoxin and are found to be between 30 to 100 nm in size with a Zeta-potential of > 25mV, display greater than 90 % mRNA encapsulation and < 1 EU/ml of endotoxin.

The accordingly obtained mRNA-LNP formulations are stored at -80 °C until further in vitro or in vivo use.

### Example 2: Lung-specific delivery of mRNA in a mouse study

Compositions as prepared in Examples 1 are used in a mouse study as formulations. The mRNA contained in the compositions code for luciferase.

The formulations are administered intravenously through bolus tail-vein injection at a dose of 1 mg mRNA / kg body weight, respectively. Four hours after injection, mice are sacrificed and tissue samples from organs (e.g., liver, lung, spleen, heart, brain, kidney) collected. Luciferase activity in tissue samples is measured by a Luciferase Assay System according to the supplier's protocol (Promega GmbH, Walldorf, Germany).

Luciferase activity was significantly increased in lung tissue compared to spleen tissue, heart tissue, brain tissue, kidney tissue and liver tissue.

### Example 3: Vasculature-specific delivery of mRNA in a cynomolgus study

A single intravenous infusion of a mRNA (PTX_RNA) which is coding for human COMP-Angiopoitin-1 formulated within the lipid system β-(L-Arginyl)-L-2,3-diamino propionic acid-N-palmityl-N-oleyl-amide / DPhyPE / mPEG-2000-DSPE (50 mol% : 49 mol% : 1 mol%) prepared as described in Example 1, is administered at different doses to cynomolgus monkeys (4 monkeys per dose group, 1 hour infusion). Blood samples are taken at pre-dose and post-dose time points (pre-dose: day 1, day 0, post-dose: t = 0.5 h, 1 h, 2 h, 4 h, 8 h, 24 h, 48 h) and aliquoted for pharmacodynamic (PD) and cytokine analysis.

Angiopoietin 1 and Angiopoietin2 levels at those different time points are measured by a standardized and commercially available ELISA assay or by a custom-made ELISA detecting the secreted protein of interest. The corresponding pharmacodynamics parameter are analyzed.

The secreted protein can be measured in a dose and time dependent manner in serum samples of the treated animals. The expression starts at 2 h post infusion and a peak of expression is reached around 6-10 h. The clearance of the protein is dependent on the serum half-life of the secreted protein and/or on the stability of the protein.

This data indicate that the formulation of the invention is a suitable mRNA formulation for protein expression in the vasculature of non-human primates (NHPs) and humans.

The features of the present invention disclosed in the specification, the claims and/or the examples may both separately and in any combination thereof be material for realizing the invention in various forms thereof.

## Claims

1. A composition comprising a lipid composition, wherein the lipid composition comprises a cationic lipid, a neutral lipid and a shielding lipid,
wherein the cationic lipid is selected from the group consisting of β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-Di-O-octadecenyl-3-trimethylammonium propane (preferably chloride salt)) and DC-cholesterol (3B-[N-(N',N'-Dimethylaminoethane)-carbamoyl]cholesterol),
wherein the neutral lipid is
a zwitterionic phospholipid selected from the group consisting of Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Dilauroyl-sn-glycero-3-phosphoethanolamine), DMPE ((1,2-Dimyristoyl-sn-glycero-3-phosphoethanolamine)), POPE ((1-Palmitoyl-2-oleoyl-sn-glycero-3-phosphoethanolamine)) and DSPE (1,2-Distearoyl-sn-glycero-3-phosphoethanolamine),
wherein the shielding lipid is a PEGylated lipid selected from the group consisting methoxyPEG-DSPE ((1,2-Distearoyl-sn-glycero-3 -phosphoethanolamine-N-[methoxy(polyethylene glycol)] (ammonium or sodium salt), methoxyPEG-DLG (1,2-Dilauroyl-rac-glycero-3-methoxypolyethylene glycol), methoxyPEG-DMG (1,2-Dimyristoyl-rac-glycero-3-methoxypolyethylene glycol), methoxyPEG-DPG (1,2-Dipalmitoyl-rac-glycero-3-methoxypolyethylene glycol), methoxyPEG-DSG (1,2-Distearoyl-rac-glycero-3-methoxypolyethylene glycol), methoxyPEG-c-DMA (N-[(Methoxy poly(ethylene glycol))carbamyl]-1,2-dimyristyloxlpropyl-3-amine), methoxyPEG-C8-ceramide (N-Octanoyl-sphingosine-1-{succinyl [methoxy(polyethylene glycol)]}), methoxyPEG-C 16-ceramide (N-Palmitoyl-sphingosine-1-{succinyl[methoxy(polyethylene glycol)]}), and
wherein the composition comprises an mRNA.

2. A composition comprising a lipid composition, wherein the lipid composition comprises a cationic lipid, a neutral lipid and a shielding lipid,
wherein the cationic lipid is selected from the group consisting of β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-Di-O-octadecenyl-3-trimethylammonium propane (chloride salt)) and DC-cholesterol (3B-[N-(N',N'-Dimethylaminoethane)-carbamoyl]cholesterol),
wherein the neutral lipid is
a zwitterionic phospholipid selected from the group consisting of Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Dilauroyl-sn-glycero-3-phosphoethanolamine), DMPE ((1,2-Dimyristoyl-sn-glycero-3-phosphoethanolamine)), POPE ((1-Palmitoyl-2-oleoyl-sn-glycero-3-phosphoethanolamine)) and DSPE (1,2-Distearoyl-sn-glycero-3-phosphoethanolamine), and
wherein the shielding lipid is a PEGylated lipid selected from the group consisting methoxyPEG-DSPE ((1,2-Distearoyl-sn-glycero-3-phosphoethanolamine-N-[methoxy(polyethylene glycol)] (ammonium or sodium salt), methoxyPEG-DLG (1,2-Dilauroyl-rac-glycero-3-methoxypolyethylene glycol), methoxyPEG-DMG (1,2-Dimyristoyl-rac-glycero-3-methoxypolyethylene glycol), methoxyPEG-DPG (1,2-Dipalmitoyl-rac-glycero-3-methoxypolyethylene glycol), methoxyPEG-DSG (1,2-Distearoyl-rac-glycero-3-methoxypolyethylene glycol), methoxyPEG-c-DMA (N-[(Methoxy poly(ethylene glycol))carbamyl]-1,2-dimyristyloxlpropyl-3-amine), methoxyPEG-C8-ceramide (N-Octanoyl-sphingosine-1-{succinyl[methoxy(polyethylene glycol)]}), methoxyPEG-C16-ceramide (N-Palmitoyl-sphingosine-1-{succinyl[methoxy(polyethylene glycol)]}), and
wherein the lipid composition forms particles, wherein the particle size is from 30 nm to 100 nm, preferably from about 30 nm to about 60 nm.

3. The composition of claim 2, wherein the composition comprises an mRNA.

4. The composition of claim 1, wherein the lipid composition forms particles, wherein the particle size is from about 30 nm to about 100 nm, preferably the particle size is from about 30 nm to about 60 nm.

5. The composition of any one of claims 2 to 4, wherein the particles comprise an mRNA

6. The composition of any one of claims 1 to 5, wherein the zeta potential of the particles is about + 25 mV to about + 80 mV, preferably the zeta potential of the particles is about + 30 mV to about + 60 mV.

7. The composition of any one of claims 1 and 3 to 6, wherein the mRNA forms part of the particles.

8. The composition of any one of claims 1 and 2 to 7, wherein the ratio of the mass of the total lipid content of the composition to the mass of the mRNA of the composition is from about 2 to about 50, preferably from about 5 to 40, and more preferably from about 10 to about 30.

9. The composition of any one of claims 1 to 8, wherein the composition is suitable for delivering a therapeutically active agent to vasculature, preferably pulmonary vasculature, more preferably to human pulmonary vasculature.

10. The composition of any one of claims 1 to 9, wherein the therapeutically active agent is an mRNA, preferably the therapeutically active agent is the mRNA comprised by the composition, more preferably the mRNA forms a complex with the lipid composition.

11. The composition of any one of claims 1 to 10, wherein the molar ratio of the lipids in the lipid composition is
- from about 20 mol-% to about 80 mol-% of the cationic lipid,
- from about 10 mol-% to about 70 mol-% of the neutral lipid, and
- from about 1 mol-% to 10 mol-% of the PEGylated lipid,
wherein the overall lipid content is 100 %,
preferably the molar ratio of the lipids in the lipid composition is
- from about 35 mol-% to about 65 mol-% of the cationic lipid,
- from about 35 mol-% to about 65 mol-% of the neutral lipid, and
- from about 1 mol-% to 5 mol-% of the PEGylated lipid,
wherein the overall lipid content is 100 %.

12. The composition of any one of claims 1 to 11, wherein the molar ratio of the lipid composition is
- 50 mol-% of the cationic lipid, wherein the cationic lipid is β-(L-Arginyl)-L-2,3-diamino propionic acid-N-palmityl-N-oleyl-amide or L-Arginyl-β-alanine-N-palmityl-N-oleyl-amide,
- 49 mol-% of the neutral lipid, wherein the neutral lipid is Diphytanoyl-PE, and
- 1 mol-% of the shielding lipid, wherein the shielding lipid is mPEG-2000-DSPE (1,2-Distearoyl-sn-glycero-3-phosphoethanolamine-N-[methoxy(polyethylene glycol)-2000] (sodium salt)).

13. The composition of any one of claims 1 to 12 for use in a method for the treatment and/or prevention of a disease, preferably, the disease is selected from the group comprising acute respiratory distress syndrome, acute lung injury, lung cancer, pulmonary metastasis, pulmonary hypertension and pulmonary artery hypertension.

14. A pharmaceutical composition comprising a composition of any one of claims 1 to 12 and a pharmaceutically active agent.

15. A method for preparing a composition of any one of claims 1 to 12, wherein the method comprises mixing a solution comprising the lipid components of the lipid composition with a solution comprising the mRNA, wherein the mixing is an in-line mixing, preferably the mixing is a non-turbulent and diffusion-based mixing, preferably a rapid non-turbulent and diffusion-based mixing.

16. The method of claim 15, wherein the mixing is a microfluidic mixing, preferably a microfluidic mixing using a microfluidic mixing device.
